# EUROPÄISCHE PATENTSCHRIFT

(11) **EP 0 481 370 B1**
(45) Veröffentlichungstag und Bekanntmachung des Hinweises auf die Patenterteilung: **28.02.1996**
(21) Anmeldenummer: 91117351.6
(22) Anmeldetag: 11.10.1991
(51) Int. Cl.: C08K 5/5373

(54) **Verwendung von Phosphonsäurediglycidylestern als Vernetzer bei Hydrogelen**
Use of phosphonic acid diglycidylesters as cross-linking agent in hydrogels
Utilisation d'esters diglycidyliques d'acide phosphonique pour la réticulation d'hydrogels

(30) Priorität: 18.10.1990 DE 4033007
(43) Veröffentlichungstag der Anmeldung: 22.04.1992
(73) Patentinhaber: HOECHST AKTIENGESELLSCHAFT, D-65926 Frankfurt am Main (DE)
(72) Erfinder: Engelhardt, Fritz, Dr., W-6000 Frankfurt am Main 60 (DE); Riegel, Ulrich, W-6000 Frankfurt am Main 60 (DE); Kleiner, Hanss-Jerg, Dr., W-6242 Kronberg 2 (DE); Müller, Wolf-Dieter, Dr., W-6238 Hofheim 3 (DE)

(56) Entgegenhaltungen:
- US-A- 2 856 369

## Beschreibung

Die vorliegende Erfindung betrifft die Verwendung von Phosphonsäurediglycidylestern als Vernetzer bei Hydrogelen, sowie Hydrogele und ihre Verwendung.

Bei der Herstellung von Hydrogelen in wäßriger Lösung werden als Vernetzer üblicherweise wasserlösliche Verbindungen wie beispielsweise Methylenbisacrylamid, Bisacrylamidoessigsäure oder Alkenylphosphon- und phosphinsäureester, aber auch schwer wasserlösliche Verbindungen wie beispielsweise Trimethylolpropantri(meth)acrylat oder Tetraallyloxyethan eingesetzt.

Aufgabe vorliegender Erfindung ist es, wasserlösliche, als Vernetzer wirkende Verbindungen bereitzustellen, durch deren Verwendung Hydrogele mit verbesserten Eigenschaften hinsichtlich Gelstärke und Wasserrückhaltevermögen erhalten werden.

Diese Aufgabe wird überraschenderweise gelöst durch die Verwendung von Verbindungen der allgemeinen Formel I 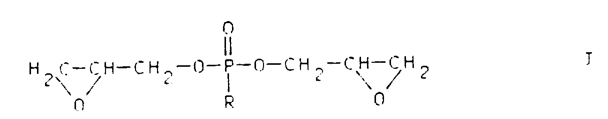 worin R Alkyl, Alkenyl oder Aryl, die gegebenenfalls substituiert sein können, bedeutet, als Vernetzer bei Hydrogelen.

Bevorzugt ist die Verwendung von Verbindungen der allgemeinen Formel I 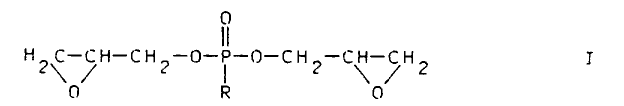 worin R (C₁-C₆)-Alkyl; (C₃-C₈)-Cycloalkyl; eine Gruppe der allgemeinen Formel II 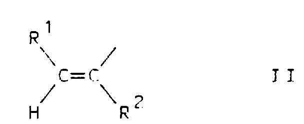 wobei R¹ und R unabhängig voneinander für Wasserstoff oder (C₁-C₄)-Alkyl stehen; oder eine Gruppe der allgemeinen Formel III 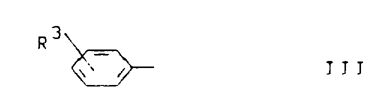 wobei R³ für Wasserstoff, Halogen oder (C₁-C₄)-Alkyl steht, bedeutet.

Alkylgruppen können geradkettig oder verzweigt sein. Für R stehendes (C₁-C₆)-Alkyl bedeutet insbesondere Methyl, Ethyl, n-Propyl, i-Propyl, n-Butyl, i-Butyl, n-Pentyl, i-Pentyl, n-Hexyl oder i-Hexyl, wobei aber (C₁-C₃)-Alkyl besonders bevorzugt ist.

Ein besonders bevorzugtes (C₃-C₈)-Cycloalkyl ist Cyclohexyl.

Für R¹ bzw. R stehendes (C₁-C₄)-Alkyl ist besonders bevorzugt Methyl. R¹ und R sind aber ganz besonders bevorzugt Wasserstoff.

Der Rest R³ kann in 2-, 3- oder 4-Position bezogen auf die Kohlenstoff-Phosphor-Bindung stehen.

Für R³ stehendes (C₁-C₄)-Alkyl bedeutet insbesondere Methyl, Ethyl, n-Propyl, i-Propyl, n-Butyl, i-Butyl oder tert.-Butyl. Für R³ stehendes Halogen bedeutet insbesondere Fluor, Chlor, Brom oder Iod.

R³ bedeutet besonders bevorzugt Methyl oder Chlor, ganz besonders bevorzugt Wasserstoff.

Die Verbindungen der allgemeinen Formel I können nach bekannten Methoden hergestellt werden.

So ist beispielsweise in der US-2.856.369 die Umsetzung von Phosphonsäurediallylestern mit Persäuren zu den entsprechenden Phosphonsäurediglycidylestern der allgemeinen Formel I beschrieben, in Doklady Akad. SSSR, 155 (1964) 1137 die Umsetzung von Phosphonigsäuredichloriden mit Glycid in Gegenwart von Base, zu den entsprechenden Phosphonigsäurediglycidylestern, die ihrerseits mit Oxidationsmitteln wie N₂O₄ in Verbindungen der allgemeinen Formel I überführt werden können.

Bevorzugt werden die Verbindungen der allgemeinen Formel I hergestellt durch Umsetzung der entsprechenden Phosphonsäuredichloride mit Glycid in Gegenwart von Base. Die Base ist notwendig zum Abfangen des bei der Reaktion von Phosphonsäuredichlorid mit Glycid entstehenden HCl, das zu Neben- bzw. Folgereaktionen führt. In Zh. Obshch. Khim 116 (1984) 2404 wird als Base NaH empfohlen, häufiger genutzt werden jedoch stickstoffhaltige Basen. Hierzu zählen insbesondere tertiäre Amine wie Trimethylamin, Triethylamin, Tripropylamin oder Tributylamin. In US-2.856.369 wird auch die Verwendung von Pyridin empfohlen. Bevorzugt eingesetzt werden Trialkylamine, besonders bevorzugt Triethylamin.

Bevorzugte Lösemittel für diese Reaktionen sind Diethylether, Methyl-tert.-butylether, Benzol, Toluol oder Xylole, aber auch andere inerte Lösemittel sowie Mischungen verschiedener Lösemittel sind geeignet. Für technische Zwecke besonders geeignet sind Lösemittel wie Methyl-tert.-butylether, Tetrahydrofuran, Toluol oder Xylole sowie deren Mischungen, besonders bevorzugt wird Toluol eingesetzt.

Die Reaktanten sowie die erforderliche Base werden üblicherweise in stöchiometrischen Mengen eingesetzt, jedoch können auch Überschüsse an Base und/oder Glycid von Vorteil sein. Gründe für die jeweils bevorzugten Einsatzmengen können verfahrenstechnischer oder anwendungstechnischer Natur sein. Unter anwendungstechnische Gründe fallen u.a. Reinheitskriterien wie: Farbe, möglichst hohe Gehalte an Wirksubstanz, möglichst geringe Gehalte an Nebenprodukten, möglichst geringe Gehalte an Ausgangsverbindungen sowie möglichst geringe Gehalte an verseifbarem und/oder ionogenem Chlor. In letzterem Fall empfiehlt sich häufig ein geringer, 1-20 Mol-%, bevorzugt 1-5 Mol-% Überschuß an Amin.

Zur Reaktion wird üblicherweise eine Mischung aus Glycid und Base im verwendeten Lösemittel vorgelegt, und das Phosphonsäuredichlorid entweder in Substanz, oder in einem der beschriebenen Lösemittel gelöst zugetropft. Auch andere Verfahrensweisen sind möglich. Beispielsweise können das Amin und das Phosphonsäuredichlorid vorgelegt werden und das Glycid zugetropft werden. Auch kontinuierliche Verfahrensweisen sind möglich. Hierzu werden z.B. Ströme aus Glycid und Amin mit einem Strom des entsprechenden Phosphonsäuredichlorides zusammengeführt und derart zur Reaktion gebracht. Einer oder beide Ströme enhält dann das erforderliche Lösemittel, das nötig ist, um Verstopfungen der Rohrleitungen durch ausfallendes Aminhydrochlorid zu vermeiden.

Nach beendeter Reaktion wird üblicherweise das ausgefallene Aminhydrochlorid abgetrennt, beispielsweise durch Filtration oder Zentrifugation. Wird ein lösemittelfreies Produkt benötigt, so wird nachfolgend das Lösemittel abtiestilliert, gegebenenfalls unter vermindertem Druck. Eine weitere Reinigung des so erhaltenen Rohproduktes kann durch Destillation unter vermindertem Druck erfolgen, entweder aus der Blase, bevorzugt jedoch in kontinuierlicher Weise durch Destillation über einen Dünnschicht- oder Kurzwegverdampfer.

Gegenstand vorliegender Erfindung sind auch wasserquellbare Hydrogele auf Basis (co)polymerisierter hydrophiler Monomere oder auf Basis natürlicher hydrophiler Polymere, dadurch gekennzeichnet, daß sie mit einer Verbindung der allgemeinen Formel I vernetzt sind.

Bei Hydrogelen auf Basis (co)polymerisierter hydrophiler Monomere können die Verbindungen der allgemeinen Formel I bereits dem zu polymerisierenden Monomerengemisch zugegeben werden. Es können aber auch die bereits polymerisierten sogenannten Pre(co)polymere nachträglich vernetzt werden. Die Vernetzung erfolgt dabei durch Reaktion der Epoxidgruppen der Verbindungen der allgemeinen Formel I mit den reaktiven Gruppen, beispielsweise -COOH, -OH oder -NHR, der Monomeren bzw. der (Co)polymeren.

Die Verbindungen der allgemeinen Formel T werden vorzugsweise in Mengen von 0,05 bis 10 Gew.%, bezogen auf das Gesamtmonomerengewicht, bzw. das Gesamtpolymergewicht eingesetzt.

Geeignete, mit Verbindungen der allgemeinen Formel T zu erfindungsgemäßen Hydrogelen vernetzbare natürliche Polymere können sowohl in unveredelter als auch in veredelter Form eingesetzt werden.

Besonders geeignet sind insbesondere Polysaccharide, wie zum Beispiel Guar, Carboxymethylhydroxypropyl-Guar, Stärke, Cellulose, Hydroxyethylcellulose sowie Alginate.

Als copolymerisierbare hydrophile Monomere kommen insbesondere Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemische davon in Frage. Bevorzugt ist Acrylsäure und deren Salze.

Die Polymerisation kann in homogener Phase z.B. in wäßriger Lösung als sog. Gelpolymerisation durchgeführt werden. Eine weitere Möglichkeit zur Synthese der erfindungsgemäßen Hydrogele bietet die Fällungspolymerisation aus organischen Lösungsmitteln wie zum Beispiel Alkoholen, bevorzugt tert. Butanol, oder Kohlenwasserstoffen wie Hexan oder Cyclohexan.

Die Polymerisation kann durch Radikalbildner wie zum Beispiel organische oder anorganische Peroxide sowie Azoverbindungen ausgelöst werden. Beispiele sind Benzoylperoxid, tert. Butylhydroperoxid, Cumolhydroperoxid, peroxid, tert. Butylhydroperoxid, Cumolhydroper, (NH₄)₂S₂O₈, K₂S₂O₈, H₂S₂O₈, H₂O₂ oder Azo-diisobutyronitril. Auch Redoxsysteme eignen sich in hervorragender Weise als Polymerisationsinitiatoren.

Die Polymerisation kann schließlich auch durch energiereiche Strahlung ausgelöst werden.

Wird die Verbindung der allgemeinen Formel I erst einem unvernetzten Pre-(Co)polymeren zugegeben, so geschieht dies in der Regel vor der Trocknung durch homogenes Vermischen, beispielsweise durch Verkneten eines wäßrigen Polymergels in einem Kneter. Auch Aufsprühen aus verdünnter Lösung auf ein Polymerpulver ist möglich, ein Nachtempern ist dabei nicht zwingend notwendig, beschleunigt aber die Vernetzungsreaktion.

Die erfindungsgemäßen Hydrogele eignen sich in hervorragender Weise als Absorbentien für wäßrige Flüssigkeiten, zur Formulierung kosmetischer Zubereitungen, als Verfestiger und/oder Binder von reaktive Gruppen enthaltenden faserigen Flächengebilden sowie als Bohrspülungen und Zementschlämme bei der Erdölgewinnung.

Für die Verwendung als sogenannte "Super Absorbing Polymers" (SAP) zum Einsatz in Hygieneartikeln, beispielsweise Windeln, Tampons oder Damenbinden, eignen sich insbesondere erfindungsgemäße Hydrogele auf Basis Acrylsäure, wobei diese teilweise als Alkali- oder Ammoniumsalz vorliegen kann. Die Neutralisation kann dabei sowohl vor als auch nach der Polymerisation erfolgen.

Auch erfindungsgemäße Hydrogele auf Basis von Polysacchariden eignen sich zum Einsatz als SAP in hervorragender Weise.

Da die Verbindungen der allgemeinen Formel I sowohl in Wasser als auch in organischen Lösungsmitteln völlig löslich sind, können erfindungsgemäße wasserquellbare Hydrogele mit gegenüber Verbindungen des Standes der Technik homogenerem Netzwerk erzielt werden. Dadurch weisen die Hydrogele neben hoher Absorptionskapazität auch hohe Gelstärke auf.

Durch Nachvernetzung mit Verbindungen der allgemeinen Formel T von bereits vorvernetzten Polymeren, die für den Einsatz als SAP vorgesehen sind, kann deren Performance hinsichtlich Absorption unter Druck wesentlich verbessert werden.

Ein weiterer Vorteil der Verbindungen der allgemeinen Formel T ist deren biologische Abbaubarkeit.

### Beispiel 1

### Synthese von Methanphosphonsäurediglycidylester (IV)

940 g Toluol werden auf -5°C gekühlt und nachfolgend 148 g (2,0 mol) Glycid und 213 g (2,1 mol) Triethylamin zugegeben. Unter lebhaftem Rühren werden 133 g (1,0 mol) Methanphosphonsäuredichlorid gelöst in 60 g Toluol, innerhalb von 1 bis 2 Stunden, bei einer Innentemperatur zwischen -5 und 0°C, zugetropft. Es wird 15 Stunden bei 0°C nachgerührt und anschließend das gebildete Triethylaminhydrochlorid abgesaugt. Es wird mit Toluol nachgewaschen und das Filtrat durch Destillation im Vacuum vom Toluol befreit. Der Rückstand wird über einen Kurzwegverdampfer bei 1 mbar und einer Badtempertatur von 175°C destilliert. Man erhält 198 g Methanphosphonsäurediglycidylester, entsprechend einer Ausbeute von 95 %. Durch einen Destillationsversuch wird der Siedepunkt bestimmt: 114-115°C/0.1 mbar. Das Produkt fällt als Diastereomerengemisch an.

| C₇H₁₃O₅P (208.2) | | | |
|---|---|---|---|
| ber.: | 40.38 % C | 6.29 % H | 14.88 % P |
| gef.: | 40.4 % C | 6.1 % H | 14.7 % P |

### Beispiel 2

### Synthese von Propanphosphonsäurediglycidylester (V)

1000 g Toluol werden auf -5°C gekühlt und nachfolgend 148 g (2.0 mol) Glycid und 213 g (2.1 mol) Triethylamin zugegeben. Unter lebhaftem Rühren werden 161 g (1 mol) Propanphosphonsäuredichlorid (technisches Isomerengemisch, bestehend aus ca. 95 % n-Propyl- und ca. 5 % iso-PropylIsomeren) innerhalb von 1 bis 2 Stunden, bei einer Innentemperatur zwischen -5 und 0°C zugetropft. Es wird 15 Stunden bei 0°C nachgerührt und anschließend das gebildete Triethylaminhydrochlorid abgesaugt. Es wird mit Toluol nachgewaschen und das Filtrat durch Destillation im Vacuum von Toluol befreit. Der Rückstand wird über einen Kurzwegverdampfer bei 1 mbar und einer Badtemperatur von 185°C destilliert. Man erhält 227 g Propanphosphonsäurediglycidylester, entsprechend einer Ausbeute von 95 %. Durch einen Destillationsversuch wird der Siedepunkt bestimmt: 115-124°C / 0.04 mbar. Das Produkt fällt als Diastereomerengemisch an.

| C₉H₁₇O₅P (236.2) | | | |
|---|---|---|---|
| ber.: | 45.77 % C | 7.25 % H | 13.1 % P |
| gef.: | 45.5 % C | 7.3 % H | 13.0 % P |

### Beispiel 3

### Synthese von Vinylphosphonsäurediglycidylester (VI)

1000 g Toluol werden auf -5°C gekühlt und nachfolgend 148 g (2.0 mol) Glycid und 213 g (2.1 mol) Triethylamin zugegeben. Unter lebhaftem Rühren werden 145 g (1.0 mol) Vinylphosphonsäuredichlorid innerhalb von 1-2 Stunden, bei einer Innentemperatur zwischen -5 und 0°C zugetropft. Es wird 15 Stunden bei 0°C nachgerührt und anschließend das gebildete Triethylaminhydrochlorid abgesaugt. Es wird mit Toluol nachgewaschen und das Filtrat durch Destillation im Vacuum vom Toluol befreit. Der Rückstand wird über einen Kurzwegverdampfer bei 0.1 mbar und einer Badtemperatur von 150°C destilliert. Man erhält 203 g Vinylphosphonsäurediglycidylester, entsprechend einer Ausbeute von 92 %. Das Produkt fällt als Diastereomerengemisch an.

| C₈H₁₃O₅P: (220.2) | | | |
|---|---|---|---|
| ber.: | 43.64 % C | 5.05 % H | 14.07 % P |
| gef.: | 43.7 % C | 6.0 % H | 13.9 % P |

### Beispiel 4

### Synthese von Benzolphosphonsäurediglycidylester (VII)

1000 g Toluol werden auf -5°C gekühlt und nachfolgend 148 g (2.0 mol) Glycid und 213 g (2.1 mol) Triethylamin zugegeben. Unter lebhaftem Rühren werden 195 g (1.0 mol) Benzolphosphonsäuredichlorid innerhalb von 1-2 Stunden, bei einer Innentemperatur zwischen -5 und 0°C zugetropft. Es wird 15 Stunden bei 0°C nachgerührt und anschließend das gebildete Triethylaminhydrochlorid abgesaugt. Es wird mit Toluol nachgewaschen und das Filtrat durch Destillation im Vacuum vom Toluol befreit. Der Rückstand wird über einen Kurzwegdampfer bei 0,15 mbar und einer Badtemperatur von 200°C destilliert. Man erhält 251 g Benzolphosphonsäurediglycidylester, entsprechend einer Ausbeute von 93 %. Durch einen Destillationsversuch wird der Siedepunkt bestimmt: 170-175°C / 0.1 mbar.

| C₁₂H₁₅O₅P: (270.2) | | | |
|---|---|---|---|
| ber. | 53,34 % C | 5.60 % H | 11.46 % P |
| gef.: | 51.1 % C | 5.6. % H | 11.3 % P |

### Beispiel 5

In einem durch geschäumtes Kunststoffmaterial gut isolierten Polyethylengefäß mit einem Fassungsvermögen von 10 l werden 4780 g E-Wasser vorgelegt, 1696 g Natriumbicarbonat darin suspendiert und langsam 1994 g Acrylsäure so zudosiert, daß ein Überschäumen der Reaktionslösung vermieden wird, wobei sich diese auf eine Temperatur von ca. 5-3^{o}C abkühlt. Es werden nun 6 g der Verbindung V (R = C₃H₇), hergestellt gemäß Beispiel 2 und 10 g eines Natrium-Diisooctylsulfosuccinates (Rewopol V 2133 der Firma REWO, Steinau) zugegeben. Bei einer Temperatur von 4^{o}C werden die Initiatoren, ein Redoxysystem, bestehend aus 2,2 g 2,2'-Azobisamidinopropan-dihydrochlorid, gelöst in 20 g E-Wasser, 4 g Kaliumperoxodisulfat, gelöst in 150 g E-Wasser sowie 0,4 g Ascorbinsäure, gelöst in 20 g E-Wasser nacheinander zugegeben und verrührt. Die Reaktionslösung wird daraufhin ohne Rühren stehengelassen, wobei durch einsetzende Polymerisation, in dessen Verlauf die Temperatur bis auf ca. 89^{o}C ansteigt, ein festes Gel entsteht. Dieses wird anschließend mechanisch zerkleinert, bei Temperaturen über 80^{o}C getrocknet und gemahlen.

Das vorliegende beschriebene Produkt wurde in herkömmlicher Weise in eine Babywindel eingearbeitet und zeichnete sich hier durch eine besonders gute Flüssigkeitsretention aus.

### Beispiel 6

Es wird vollkommen analog Beispiel 5 verfahren, nur werden jetzt 6,0 g der Verbindung VI (R = CH=CH₂), hergestellt gemäß Beispiel 3, eingesetzt. Auch das hier resultierende Produkt ist hervorragend für den Einsatz in Babywindeln geeignet und zeichnet sich durch gute Flüssigkeitsretention aus.

### Beispiel 7

Unter adiabatischen Bedingungen werden in einem 1,5 l zylindrischen Weithalsreaktionskolben 1287 g auf 15^{o}C abgekühltes E-Wasser vorgelegt und 255 g Acrylsäure sowie 1,28 g Tetraallyloxyethan darin gelöst. Es wird Stickstoff in die Monomerlösung eingeleitet (ca. 2 l/Min. für ca. 20 Min.), um den Sauerstoffgehalt zu erniedrigen. Bei einem Gehalt von 1,5 ppm O₂ werden 7,7 g einer 10%igen wäßrigen Lösung von 2,2'-Azobis(2-amidinopropan)-dihydrochlorid zugegeben, nach weiterem N₂-Einleiten und einem O₂-Gehalt von 1,3 ppm werden 2,6 g einer 1 %igen H₂O₂-Lösung zugegeben und schließlich bei einem O₂-Gehalt von 1,0 ppm werden 6,4 g einer 0,1 %igen Ascorbinsäurelösung zugegeben. Durch einsetzende Polymerisation, in deren Verlauf die Temperatur bis auf ca. 65^{o}C ansteigt, entsteht ein festes Gel, das anschließend mechanisch zerkleinert wird. 400 g des zerkleinerten Gels werden mit 56,5 g Natronlauge 50%ig versetzt (Neutralisationsgrad der Acrylsäure 74 Mol-%), zweimal durchgeknetet, mit 25 g einer 1%igen wäßrigen Lösung der Verbindung V (R = C₃H₇), hergestellt gemäß Beispiel 2 versetzt, wiederum zweimal verknetet, anschließend bei Temperaturen über 150^{o}C in dünner Schicht getrocknet, gemahlen und gesiebt.

Man erhält ein Produkt, im wesentlichen u.a. gekennzeichnet durch folgende physikalischen Daten, alle gemessen in NaCl 0,9 %. Extrahierbare Anteile (1 h-Wert) 2,1 %, Absorption unter Druck (20 g/cm) = 29,8 g/g.

### Beispiel 8

Zu 300 g eines zerkleinerten zu 73 Mol-% neutralisierten 30%igen Polymergels mit einer Monomerenzusammensetzung von 99,7 Gew.-% Acrylsäure und 0,3 Gew.-% Triallylamin, hergestellt analog Beispiel 5, werden 0,23 g der Verbindung IV (R = CH₃), hergestelt gemäß Beispiel 1, gelöst in 40 g Wasser, gegeben, homogen verknetet, zerkleinert, bei 180^{o}C bis zu einer Restfeuchte von 3 % getrocknet, gemahlen und gesiebt.

### Beispiel 9

Es wird vollkommen analog zu Beispiel 8 verfahren, nur werden 0,46 g der Verbindung IV (R = CH₃), hergestellt gemäß Beispiel 1, eingesetzt.

### Beispiel 10

Es wird vollkommen analog zu Beispiel 8 verfahren, nur werden 0,23 g der Verbindung V (R = C₃H₇), hergestellt gemäß Beispiel 2, eingesetzt.

### Beispiel 11

Es wird vollkommen analog zu Beispiel 8 verfahren, nur werden 0,46 g der Verbindung V (R = C₃H₇), hergestellt gemäß Beispiel 2, eingesetzt.

### Beispiel 12

Es wird vollkommen analog zu Beispiel 8 verfahren, nur werden 1,00 g der Verbindung VII (R = Phenyl), hergestellt gemäß Beispiel 4, eingesetzt.

Die erhaltenen Produkte der Beispiele 8 bis 12 sind durch folgende in Tabelle I zusammengefaßte Daten gekennzeichnet:

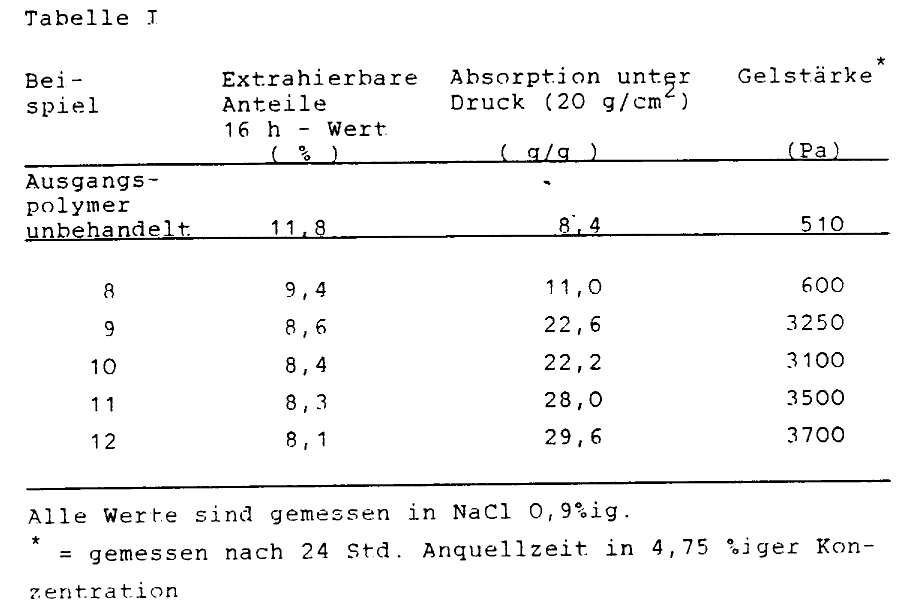

### Beispiel 13

Auf 45^{o}C aufgewärmte handelsübliche, teilneutralisierte, vernetzte Polyacrylsäure für den Einsatz als Superabsorber in Babywindeln, wird 0,2 Gew.% der Verbindung V (R = C₃H₇), hergestellt gemäß Beispiel 2, in 10%iger, wäßriger Lösung in einem PETTERSON & KELLY-Mischer aufgedüst und 10 Min. gemischt. Nach Abkühlen auf Raumtemperatur wurden folgende, in Tabelle II aufgeführten, im Vergleich zum Ausgangsprodukt verbesserten Werte gefunden:

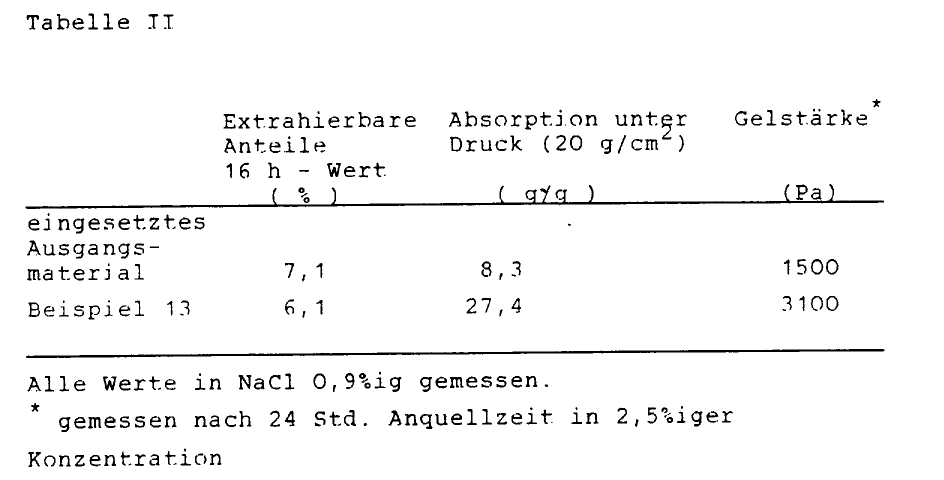

### Beispiel 14

100 g hochmolekulare, unvernetzte Polyacrylsäure mit einem Neutralisationsgrad von 53 Mol-% in Form eines zerkleinerten Gels, hergestellt analog Beispiel 5 ohne Vernetzer wird mit 120 g Guarmehl sowie 100 g 0,15 % wäßriger Lösung der Verbindung IV, hergestellt gemäß Beispiel 1, homogen verknetet, zerkleinert, im Luftstrom bei 180^{o}C 15 Min. getrocknet, gemahlen und gesiebt. Man erhält ein wasserquellbares Produkt mit einem Wasserabsorptionsvermögen vom mehrfachen des Eigengewichts.

In den Beispielen 15 bis 21, aufgeführt in Tabelle III, wird die Herstellung von wasserquellbaren Produkten mit gutem Absorptionsvermögen beschrieben durch Vernetzung von Polymeren verschiedenen Ursprungs mit erfindungsgemäßen Verbindungen, was so geschieht, daß die Polymeren in Wasser angeteigt, mit dem Vernetzer versetzt, homogen verknetet, im Luftstrom bei 180^{o}C getrocknet, gemahlen und gesiebt werden.

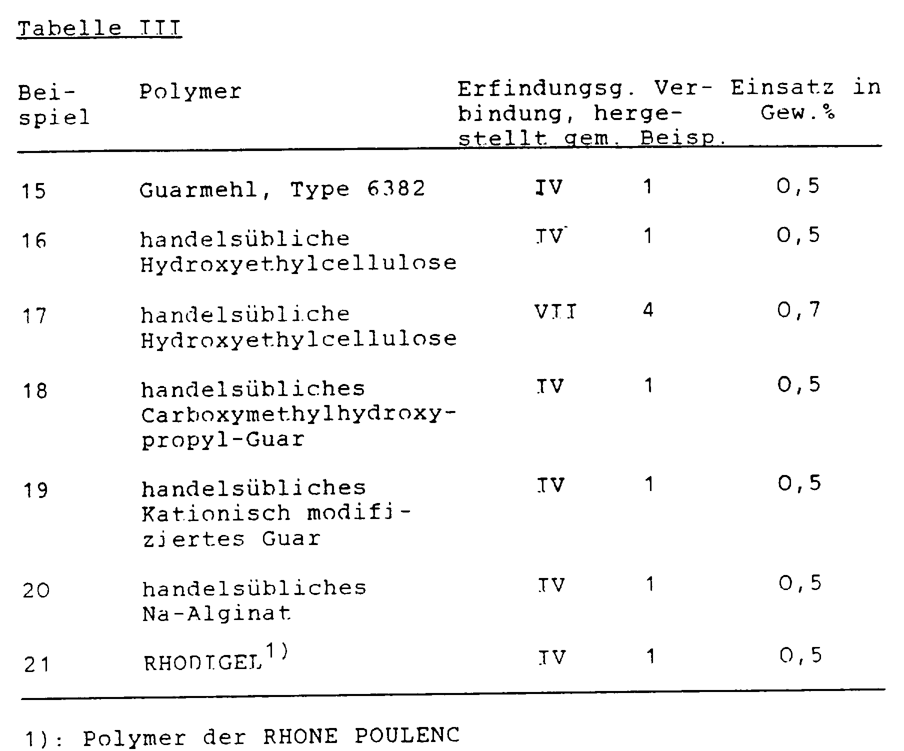

### Beispiel 22

In einem 1 l-Polymerisationskolben aus Glas, versehen mit Rührwerk, Thermometer und Rückflußkühler werden 600 ml Hexan vorgelegt und 98,9 g Acrylsäure sowie 1,1 g der Verbindung VI (R = CH=CH₂), hergestellt gemäß Beispiel 3, darin gelöst. Unter Einleiten eines schwachen N₂-Stromes wird mittels eines elektrisch beheizten Wasserbades auf 68^{o}C angeheizt, worauf die Zugabe von 1,0 g Di-Laurylperoxid erfolgt. Nach Einsetzen der Polymerisation tritt deutlicher Rückfluß ein, und das entstandene Polymer flockt aus. Es wird 3 Stunden unter Rückfluß nachgerührt, dann das Polymer abgesaugt und im Trockenschrank bis zur Gewichtskonstanz getrocknet. Man erhält 100 g eines weißen Pulvers, das als saurer Verdicker in kosmetischen Zubereitungen eingesetzt werden kann.

### Beispiel 23

In einem 1 l-Polymerisationskolben aus Glas, versehen mit Rührwerk, Thermometer, Rückflußkühler, Gaseinleitungsrohr und elektrisch beheiztem Wasserbad werden 600 ml tert.-Butanol vorgelegt und 0,1 g der Verbindung VII (R=Phenyl), hergestellt gemäß Beispiel 4, und 65 g Acrylamido-2-methyl-propansulfonsäure (AMPS) unter Rühren darin suspendiert. Über das Gaseinleitungsrohr werden nun ca. 5,5 g Ammoniakgas eingeleitet, wobei eine schwach trübe Lösung entsteht. Der pH dieser Lösung muß >7 betragen. Anschließend werden 15 g Acrylamid und 20 g N-Vinyl-N-methylacetamid zugegeben und die Lösung unter Einleiten eines schwachen N₂-Stromes auf eine Temperatur von 50^{o}C angeheizt. Es wird 1,0 g Azo-diisobutyronitril als Initiator hinzugegeben und die Rührgeschwindigkeit auf 60 - 80 UpM gedrosselt. Nach ca. 10 Min. setzt die Polymerisation ein, was an einem Ausflocken des Polymerisates sowie einem Temperaturanstieg festzustellen ist. Im Verlauf von ca. 20 Min. entsteht ein dicker Brei und die Temperatur steigt bis auf ca. 75^{o}C an. Nach Erreichen des Temperaturmaximums wird 2 Stunden bei 80^{o}C nachgerührt, das Polymer abgesaugt und im Vacuumtrockenschrank bei 60^{o}C bis zur Gewichtskonstanz getrocknet. Man erhält 105 g weißes Pulver mit einem Schüttgewicht von ca. 0,2 kg/l, das sich hervorragend als Additiv in Bohrspülungen und Zementschlämmen bei der Exploration von Erdgas und Erdöl eignet.

### Beispiel 24

Eine 10%ige wäßrige Copolymerenlösung mit der Monomerenzusammensetzung von 90 Gew% Acrylsäure und 10 Gew% Vinylphosphonsäure, mit NaOH teilneutralisiert auf einen pH-Wert von 5,5 - 6,0 und versetzt mit 1,0 Gew.% (bezogen auf Polyacrylsäure) der Verbindung V, hergestellt gemäß Beisiel 2, eignet sich für den Einsatz als Binder/Verfestiger von Vliesen, verbunden mit dem Vorteil besserer Saugleistung. Hierzu wird sie gleichmäßig beidseitig auf ein Saugkissen aus CelluloseFluff (ca. 6x20x1,5 cm/ BxLxH) so aufgesprüht, daß das Saugkissen mit 1 % des Polymerfeststoffes, bezogen auf das Trokkengewicht des Saugkissens, beaufschlagt wird. Nach 24 stündiger Lagerung bei Raumtemperatur an der Luft oder entsprechender kürzerer Verweilzeit bei höheren Temperaturen wurden die so behandelten gegen entsprechende unbehandelte Saugkissen auf Festigkeit und Saugkapazität untersucht. Die Festigkeit wurde derart geprüft, daß die Saugkissen in einem speziellen Zerwirbelungsgefäß einen definierten Luftstrom ausgesetzt waren. Die durch die Wirbelung entstandenen, vom Saugkissen abgetrennten Bestandteile wurden über ein Sieb definierter Maschenweite abgesaugt. Bestimmt wurde anschließend der Anteil an unzerstörtem, vor dem Sieb zurückgebliebenem nicht abgesaugtem Kissenmaterial, in % bezogen auf Ausgangsgewicht.

Die Saugkapazität wurde folgendermaßen bestimmt: Das Saugkissen wurde plan auf einem Siebblech liegend eine Minute in 0,9 %ige NaCl-Lösung getaucht. Anschließend wurde das Siebblech herausgeholt und eine Minute abtropfen lassen. Hierzu wurde die Versuchsanordnung etwa 45^{o}C geneigt. Es wurde die Gewichtszunahme pro Gramm Saugkissen berechnet.

In Bezug auf Festigkeit konnte bei den behandelten Saugkissen im Vergleich zu unbehandelten eine Verbesserung um ca. 20 %, in Bezug auf Saugkapazität um ca. 10 % festgestellt werden.

## Patentansprüche (Patentansprüche für folgende(n) Vertragsstaat(en): AT, BE, CH, LI, DE, DK, FR, GB, GR, IT, NL, SE)

1. Verwendung von Verbindungen der allgemeinen Formel I 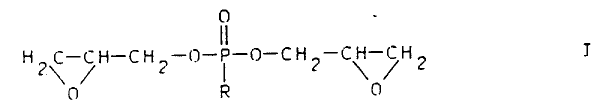 worin R Alkyl, Alkenyl oder Aryl, die gegebenenfalls substituiert sein können, bedeutet, als Vernetzer bei Hydrogelen.

2. Verwendung von Verbindungen gemäß Anspruch 1 der allgemeinen Formel 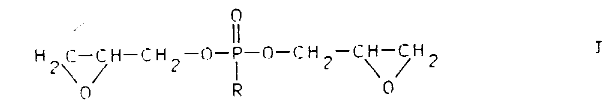 worin R (C₁-C₆)-Alkyl; (C₃-C₈)-Cycloalkyl; eine Gruppe der allgemeinen Formel II 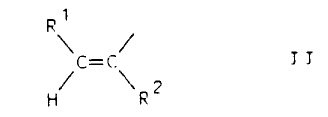 wobei R¹ und R unabhängig voneinander für Wasserstoff oder (C₁-c₄)-Alkyl stehen; oder eine Gruppe der allgemeinen Formel III 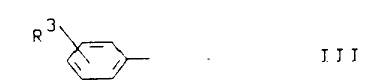 wobei R³ für Wasserstoff, Halogen oder (C₁-C₄)-Alkyl steht, bedeutet.

3. Verwendung gemäß Anspruch 1 und/oder 2, dadurch gekennzeichnet, daß für R stehendes Alkyl (C₁-C₃)-Alkyl ist.

4. Verwendung gemäß Anspruch 2, dadurch gekennzeichnet, daß in der allgemeinen Formel II R¹ und R Wasserstoff bedeuten.

5. Verwendung gemäß Anspruch 2, dadurch gekennzeichnet, daß in der allgemeinen Formel III R³ Methyl oder Chlor und ganz besonders bevorzugt Wasserstoff bedeutet.

6. Verwendung gemäß einem oder mehreren der Ansprüche 1 bis 5, dadurch gekennzeichnet, daß die Verbindungen der allgemeinen Formel I in Mengen von 0,05 bis 10 Gew.%, bezogen auf Gesamtmonomerengewicht bzw. Gesamtpolymerengewicht, eingesetzt werden.

7. Wasserquellbares Hydrogel auf Basis (co)polymerisierter hydrophiler Monomere oder auf Basis natürlicher hydrophiler Polymere, dadurch gekennzeichnet, daß es mit einer Verbindung der allgemeinen Formel I gemäß Anspruch 1 vernetzt ist.

8. Wasserquellbares Hydrogel gemäß Anspruch 7, dadurch gekennzeichnet, daß als hydrophile Monomere Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemische davon eingesetzt werden.

9. Wasserquellbares Hydrogel gemäß Anspruch 7 und/oder 8, dadurch gekennzeichnet, daß als hydrophiles Monomer Acrylsäure und/oder deren Salze eingesetzt werden.

10. Wasserquellbares Hydrogel gemäß Anspruch 7, dadurch gekennzeichnet, daß als natürliche hydrophile Polymere Polysaccharide wie zum Beispiel Guar, Carboxymethylhydroxypropyl-Guar, Stärke, Cellulose, Hydroxyethylcellulose oder Alginate eingesetzt werden.

## Patentansprüche (Patentansprüche für folgende(n) Vertragsstaat(en): ES)

1. Verwendung von Verbindungen der allgemeinen Formel I 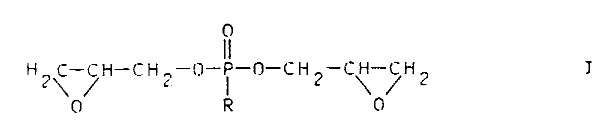 worin R Alkyl, Alkenyl oder Aryl, die gegebenenfalls substituiert sein können, bedeutet, als Vernetzer bei Hydrogelen.

2. Verwendung von Verbindungen gemäß Anspruch 1 der allgemeinen Formel 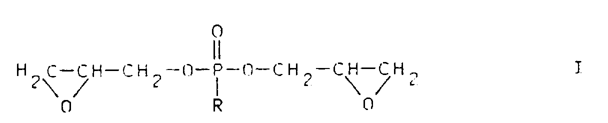 worin R (C₁-C₆)-Alkyl; (C₃-C₈)-Cycloalkyl; eine Gruppe der allgemeinen Formel II 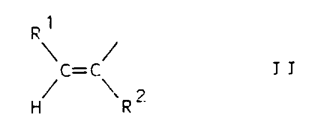 wobei R¹ und R unabhängig voneinander für Wasserstoff oder (C₁-c₄)-Alkyl stehen; oder eine Gruppe der allgemeinen Formel III 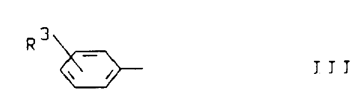 wobei R³ für Wasserstoff, Halogen oder (C₁-C₄)-Alkyl steht, bedeutet.

3. Verwendung gemäß Anspruch 1 und/oder 2, dadurch gekennzeichnet, daß für R stehendes Alkyl (C₁-C₃)-Alkyl ist.

4. Verwendung gemäß Anspruch 2, dadurch gekennzeichnet, daß in der allgemeinen Formel II R¹ und R Wasserstoff bedeuten.

5. Verwendung gemäß Anspruch 2, dadurch gekennzeichnet, daß in der allgemeinen Formel III R³ Methyl oder Chlor und ganz besonders bevorzugt Wasserstoff bedeutet.

6. Verwendung gemäß einem oder mehreren der Ansprüche 1 bis 5, dadurch gekennzeichnet, daß die Verbindungen der allgemeinen Formel I in Mengen von 0,05 bis 10 Gew.%, bezogen auf Gesamtmonomerengewicht bzw. Gesamtpolymerengewicht, eingesetzt werden.

7. Verfahren zur Herstellung wasserquellbarer Hydrogele auf Basis (co)polymerisierter hydrophiler Monomerer durch Copolymerisation oder durch nachträgliche Vernetzung von Pre(co)polymeren oder auf Basis natürlicher hydrophiler Polymerer durch nachträgliche Vernetzung, dadurch gekennzeichnet, daß eine Verbindung der allgemeinen Formel I des Anspruchs 1 als Vernetzer eingesetzt wird.

8. Verfahren gemäß Anspruch 7, dadurch gekennzeichnet, daß als hydrophile Monomere Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemische davon eingesetzt werden.

9. Verfahren gemäß Anspruch 7 und/oder 8, dadurch gekennzeichnet, daß als hydrophiles Monomer Acrylsäure und/oder deren Salze eingesetzt werden.

10. Verfahren gemäß Anspruch 7, dadurch gekennzeichnet, daß als natürliche hydrophile Polymere Polysaccharide wie zum Beispiel Guar, Carboxymethyl-hydroxypropyl-Guar, Stärke, Cellulose, Hydroxyethylcellulose oder Alginate eingesetzt werden.

## Claims (Claims for the following Contracting State(s): AT, BE, CH, LI, DE, DK, FR, GB, GR, IT, NL, SE)

1. Use of compounds of the general formula I 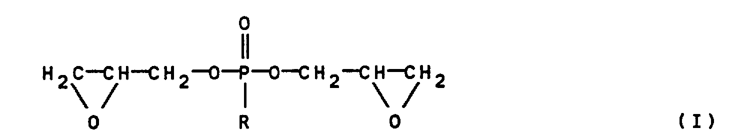 in which R denotes alkyl, alkenyl or aryl, which can be optionally substituted, as crosslinkers in the preparation of hydrogels.

2. Use of compounds according to Claim 1, of the general formula 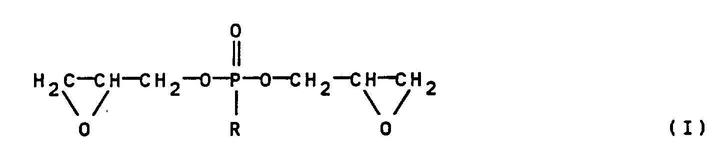 in which R denotes (C₁-C₆)-alkyl; (C₃-C₈)-cycloalkyl; a group of the general formula II 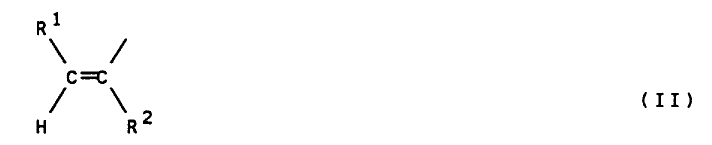 in which R¹ and R independently of one another represent hydrogen or (C₁-C₄)-alkyl; or a group of the general formula III 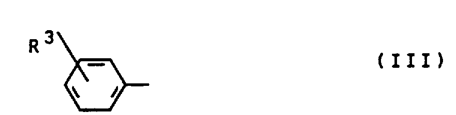 in which R³ represents hydrogen, halogen or (C₁-C₄)-alkyl.

3. Use according to Claim 1 and/or 2, characterised in that alkyl R is (C₁-C₃)-alkyl.

4. Use according to Claim 2, characterised in that in the general formula II R¹ and R denote hydrogen.

5. Use according to Claim 2, characterised in that in the general formula III R³ denotes methyl or chlorine and very particularly preferably hydrogen.

6. Use according to one or more of Claims 1 to 5, characterised in that the compounds of the general formula I are employed in amounts of 0.05 to 10% by weight, relative to the total monomer weight or total polymer weight respectively.

7. Water-swellable hydrogel based on (co)polymerised hydrophilic monomers or based on natural hydrophilic polymers, characterised in that it is crosslinked with a compound of the general formula I according to Claim 1.

8. Water-swellable hydrogel according to Claim 7, characterised in that the hydrophilic monomers employed are acrylic acid, methacrylic acid, crotonic acid, 2-acrylamido-2-methylpropanesulphonic acid and -phosphonic acid, vinylphosphonic acid, vinylphosphonic acid half-esters, their salts, acrylamide, N-vinylamides or mixtures thereof.

9. Water-swellable hydrogel according to Claim 7 and/or 8, characterised in that the hydrophilic monomer employed is acrylic acid and/or its salts.

10. Water-swellable hydrogel according to Claim 7, characterised in that the natural hydrophilic polymers employed are polysaccharides such as, for example, guar, carboxymethylhydroxypropyl guar, starch, cellulose, hydroxyethylcellulose or alginates.

## Claims (Claims for the following Contracting State(s): ES)

1. Use of compounds of the general formula I 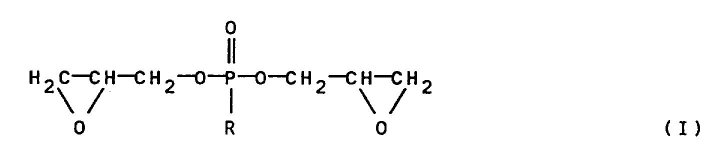 in which R denotes alkyl, alkenyl or aryl, which can be optionally substituted, as crosslinkers in the preparation of hydrogels.

2. Use of compounds according to Claim 1, of the general formula 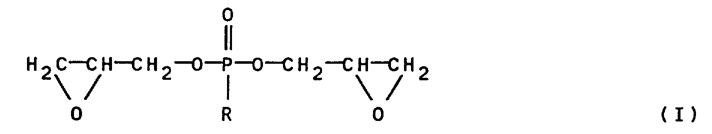 in which R denotes (C₁-C₆)-alkyl; (C₃-C₈)-cycloalkyl; a group of the general formula II 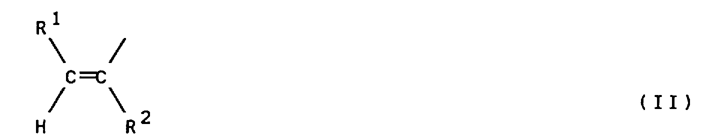 in which R¹ and R independently of one another represent hydrogen or (C₁-C₄)-alkyl; or a group of the general formula III 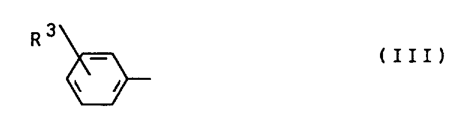 in which R³ represents hydrogen, halogen or (C₁-C₄)-alkyl.

3. Use according to Claim 1 and/or 2, characterised in that alkyl R is (C₁-C₃)-alkyl.

4. Use according to Claim 2, characterised in that in the general formula II R¹ and R denote hydrogen.

5. Use according to Claim 2, characterised in that in the general formula III R³ denotes methyl or chlorine and very particularly preferably hydrogen.

6. Use according to one or more of Claims 1 to 5, characterised in that the compounds of the general formula I are employed in amounts of 0.05 to 10% by weight, relative to the total monomer weight or total polymer weight respectively.

7. Process for preparing water-swellable hydrogels based on (co)polymerised hydrophilic monomers by copolymerisation or subsequent crosslinking of pre(co)polymers or based on natural hydrophilic polymers by subsequent crosslinking, characterised in that a compound of the general formula I according to Claim 1 is employed as crosslinker.

8. Process according to Claim 7, characterised in that the hydrophilic monomers employed are acrylic acid, methacrylic acid, crotonic acid, 2-acrylamido-2-methylpropanesulphonic acid and -phosphonic acid, vinylphosphonic acid, vinylphosphonic acid half-esters, their salts, acrylamide, N-vinylamides or mixtures thereof.

9. Process according to Claim 7 and/or 8, characterised in that the hydrophilic monomer employed is acrylic acid and/or its salts.

10. Process according to Claim 7, characterised in that the natural hydrophilic polymers employed are polysaccharides such as, for example, guar, carboxymethylhydroxypropyl guar, starch, cellulose, hydroxyethylcellulose or alginates.

## Revendications (Revendications pour l'(les) Etat(s) contractant(s) suivant(s): AT, BE, CH, LI, DE, DK, FR, GB, GR, IT, NL, SE)

1. Emploi de composés de formule générale I 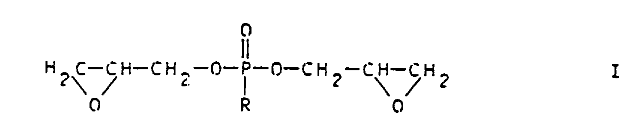 dans laquelle R désigne un alkyle, un alcényle ou un aryle, pouvant être éventuellement substitués, comme agents réticulants pour la formation d'hydrogels.

2. Emploi de composés selon la revendication 1, de formule générale 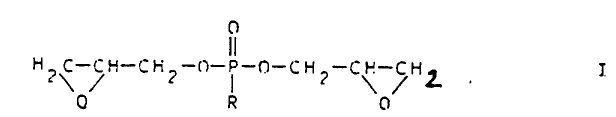 dans laquelle R désigne un alkyle en (C₁-C₆), cycloalkyle en (C₃-C₈) ; un groupe de formule générale II 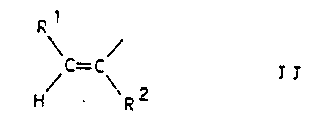 R¹ et R étant chacun, indépendamment l'un de l'autre, l'hydrogène ou un alkyle en (C₁-C₄) ; ou encore un groupe de formule générale III 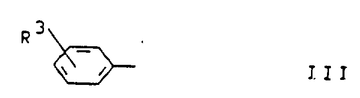 R³ désignant l'hydrogène, un halogène ou un alkyle en (C₁-C₄).

3. Emploi selon la revendication 1 et/ou 2, caractérisé en ce que R, comme alkyle, est un alkyle en (C₁-C₃).

4. Emploi selon la revendication 2, caractérisé en ce que dans la formule générale II, R¹ et R sont l'hydrogène.

5. Emploi selon la revendication 2, caractérisé en ce que dans la formule générale III R³ est le groupe méthyle ou le chlore, mais plus spécialement et de préférence l'hydrogène.

6. Emploi selon une ou plusieurs des revendications 1 à 5, caractérisé en ce que l'on utilise les composés de formule générale I dans des proportions de 0,05 à 10 % du poids total des monomères ou bien des polymères.

7. Hydrogel gonflable à l'eau à base de monomères hydrophiles (co)polymérisés ou de polymères hydrophiles naturels, hydrogel caractérisé en ce qu'il est réticulé avec un composé de formule générale I selon la revendication 1.

8. Hydrogel gonflable à l'eau selon la revendication 7, caractérisé en ce qu'on utilise comme monomères hydrophiles les acides acrylique, méthacrylique, crotonique, 2-acrylamido-2-méthylpropane-sulfonique et -phosphonique vinylphosphonique et les hémi-esters de l'acide vinylphosphonique, leurs sels, l'acrylamide, des N-vinylamides ou des mélanges de ces composés.

9. Hydrogel gonflable à l'eau selon la revendication 7 et/ou 8, caractérisé en ce qu'on utilise comme monomère hydrophile l'acide acrylique et/ou des sels de cet acide.

10. Hydrogel gonflable à l'eau selon la revendication 7, caractérisé en ce qu'on utilise des polymères hydrophiles naturels des polysaccharides par exemple le gouar, le carboxymethyl-hydroxypropyl-gouar, l'amidon, la cellulose et l'hydroxyméthylcellulose ou les alginates.

## Revendications (Revendications pour l'(les) Etat(s) contractant(s) suivant(s): ES)

1. Emploi de composés de formule générale I 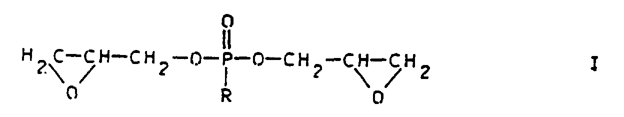 dans laquelle R désigne un alkyle, un alcényle ou un aryle, pouvant être éventuellement substitués, comme agents réticulants pour la formation d'hydrogels.

2. Emploi de composés selon la revendication 1, de formule générale 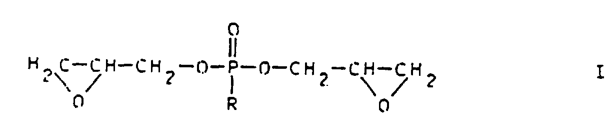 dans laquelle R désigne un alkyle en (C₁-C₆), cycloalkyle en (C₃-C₈), un groupe de formule générale II 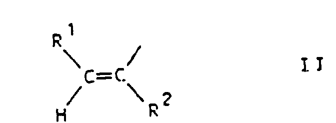 R¹ et R étant chacun, indépendamment l'un de l'autre, l'hydrogène ou un alkyle en (C₁-C₄) ou encore un groupe de formule générale III 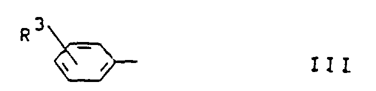 R³ désignant l'hydrogène, un halogène ou un alkyle en (C₁-C₄).

3. Emploi selon la revendication 1 et/ou 2, caractérisé en ce que R, comme alkyle, est un alkyle en (C₁-C₃).

4. Emploi selon la revendication 2, caractérisé en ce que dans la formule générale II, R¹ et R sont l'hydrogène.

5. Emploi selon la revendication 2, caractérisé en ce que dans la formule générale III R³ est le groupe méthyle ou le chlore mais plus spécialement et de préférence l'hydrogène.

6. Emploi selon une ou plusieurs des revendications 1 à 5, caractérisé en ce que l'on utilise les composés de formule générale I dans des proportions de 0,05 à 10 % du poids total des monomères ou bien des polymères.

7. Procédé de préparation d'hydrogels gonflables à l'eau à base de monomères hydrophiles (co)polymérisés par copolymérisation ou réticulation ultérieure de pré(co)polymères ou à base de polymères hydrophiles naturels par réticulation ultérieure, procédé caractérisé en ce que l'on utilise comme agent réticulant un composé de formule générale I selon la revendication 1.

8. Procédé selon la revendication 7, caractérisé en ce qu'on utilise comme monomères hydrophiles les acides acrylique, méthacrylique, crotonique, 2-acrylamido-2-méthylpropane-sulfonique, ou -phosphonique, vinylphosphonique et les hémi-esters de l'acide vinylphosphonique, leurs sels, l'acrylamide, des N-vinylamides ou des mélanges de ces composés.

9. Procédé selon la revendication 7 et 8, caractérisé en ce qu'on utilise comme monomère hydrophile l'acide acrylique et/ou des sels de cet acide.

10. Procédé selon la revendication 7, caractérisé en ce qu'on utilise des polymères hydrophiles naturels des polysaccharides, par exemple le gouar, le carboxyméthylhydroxypropyl-gouar, l'amidon, la cellulose et l'hydroxyméthylcellulose ou les alginates.
